# EUROPEAN PATENT SPECIFICATION

(11) **EP 0 666 768 B1**
(45) Date of publication and mention of the grant of the patent: **29.01.1997**
(21) Application number: 93924867.0
(22) Date of filing: 02.11.1993
(51) Int. Cl.: B01D 15/08, G01N 30/02

(54) **A LIQUID CHROMATOGRAPHIC SYSTEM**
FLÜSSIGKEITSCHROMATOGRAPHIESYSTEM
SYSTEME DE CHROMATOGRAPHIE EN PHASE LIQUIDE

(30) Priority: 02.11.1992 SE 9203222
(43) Date of publication of application: 16.08.1995
(73) Proprietor: Pharmacia Biotech AB, 751 82 Uppsala (SE)
(72) Inventor: BERGSTRÖM, Jan, S-740 22 Bälinge (SE); SÖDERBERG, Lennart, S-754 40 Uppsala (SE)
(74) Representative: Widén, Björn
(86) International application number: SE9300913
(87) International publication number: WO9409879

(56) References cited:
- EP-A- 0 078 435
- WO-A-90/06507
- WO-A-92/04958
- US-A- 4 089 207
- US-A- 4 719 011
- Orbit Search Service, File WPAT, Accession Number 87-011839/02, HITACHI PLANT ENG CONST; & JP,A,61 270 658, 86.11.29, 8702, DERWENT ABSTRACT.

## Description

### Technical Field and Background of the Invention

The present invention relates to a liquid chromatographic system which includes one or more flow lines that pass through a plurality of units (modules) having functions that can be used in chromatography. The flow lines and units may be fixedly mounted on an appropriate stand (frame), for instance a base plate.

The functional units of earlier described systems include chromatographic columns, filters, valves, branches, connections which operate to lead liquid from and to the system, injection ports, for instance for charging samples, detector connectors, and so on. In the earlier described systems, these units are mutually connected together with the aid of flexible tubings. A common feature of the known systems is that they shall enable eluants, such as buffers and solvents, for instance, to be mixed and introduced in a predetermined order and at predetermined positions.

The connection between a module and a flow line is made through an end-connecting means on the module and a connecting device on the flow line.

The systems often use small particles as the separation medium, which requires the application of high pressures, particularly when high resolution is desired. The apparatus arrangements and the various tubing connections have resulted in highly complicated "plumbing" tubing-based systems which are not easy to follow. Such systems are normally encumbered with such problems as leakage, the ingress of air and wrongly connected tubings, both when the system is connected-up and when wishing to exchange or replace a unit in the system. In order to operate these systems, it is often necessary to be an expert in chromatography and also a good instrument technician.

A module/system for chromatographic columns has previously been disclosed in US-A-4,719,011. An exchangeable column with end-connecting means and connecting devices has been described in WO-A-9204958.

### Objects of the Invention

The object of the present invention is to provide a system which is of simple construction, which can be viewed without difficulty and which includes readily exchangeable units and, in principle, will include no tubing bundles or hanks. The invention presents a new user interface in liquid chromatography and has the function of the system placed in focus. When developing the invention, particular thought has been given to the possibility of testing strategies quickly and simply in conjunction with the development of complex chromatographic methods within the framework of research and industrial separation processes, including laboratory, pilot plant and final industrial processes. Another object of the invention is to provide a teaching aid in schools and educational institutions.

### Brief Description of the Drawings

Different exemplifying embodiments of the invention are illustrated in Figures 1-16. The variant most preferred at present is illustrated in detail in Figures 1-13. Components which have analog functions are identified with the same reference signs in the various Figures.
- **Figure 1**: illustrates an embodiment which includes a base plate (1) as a base unit, and two different end-pieces (2,3), which form ancillaries. Figure 1 is a front view (with the plate placed vertically) and illustrates the basic structure.
- **Figure 2**: illustrates an alternative method of connecting the flow by means of an end-piece.
- **Figure 3**: illustrates a connector for connecting together two base plates.
- **Figure 4**: illustrates in **Fig. 4a** the base plate of Fig. 1 when seen from one side in a longitudinal plane through a flow line to which a separation module (4) is connected. **Fig. 4b** is a sectional view taken on the line A-A' of Fig. 4a. **Fig. 4c** contains two mutually perpendicular section views of the lower part (the end-connector) of a disconnected module.
- Figures 5a,b: illustrate separation modules having one and two end-connecting means respectively.
- Figure 6: illustrates a filter module.
- Figure 7: illustrates a module for connection to an outer unit.
- Figure 8a: is a side view of a flow bridge intended to conduct flow between two connecting devices.
- Figures 8b-e: illustrate variants of flow bridges, seen from above.
- Figure 9: illustrates a flow bridge with a symbol for an incorporated function (the rectangle).
- Figure 10: illustrates an example of a detector cell.
- Figures 11a-c: illustrate different forms of sample injection devices (ports and loops).
- Figures 12a,b: illustrate a variant of an injection port which is connected to a loop and to a container for surplus sample (wastes).
- Figure 13: is a template for the construction of a system configuration.
- Figures 14-16: illustrate four alternative variants of the embodiments illustrated in Figures 1-12. The variant illustrated in Figure 16 has a flow line which is defined by the flow channels and functions of connected modules. The flow line illustrated in Figures 14 and 15 also includes intermediate segments which are attached to a stand or a base plate.

### The Invention

The invention provides a liquid chromatography system as indicated in claim 1. The invention is characterized in that the modules have end-connecting means which enable them to be connected in any desired sequence to connecting devices in the flow lines of the system. The couplings provided by the end-connecting means will preferably be quick-couplings, by which is meant that the flow channel of a module can be sealingly connected to the flow line in a connecting device by a simple joining movement, optionally with a pressing, rotating or sliding manipulation. A quick-coupling will normally have a holding function. Examples of quick-couplings include bayonet couplings, snap-in couplings, slide couplings, etc. Couplings of the kind which are screwed together are not included in the term quick-coupling.

The invention resides in a chromatography system of the kind defined in the introduction and comprising:
(a) at least one flow line (5) which includes along its length two or more devices (6) (connecting devices) by means of which a module (7) having the function described below can be connected operationally; and
(b) at least one separation module and at least one further module, wherein said module or further module has one or more end-connecting means (8) which are geometrically and functionally adapted to said connecting device (6) so that modules (7) that are provided with such end-connecting means (8) can be functionally connected to any of said connecting device (6).

### AN ILLUSTRATION OF THE INVENTION WITH A STARTING POINT FROM THE FIGURES

### General

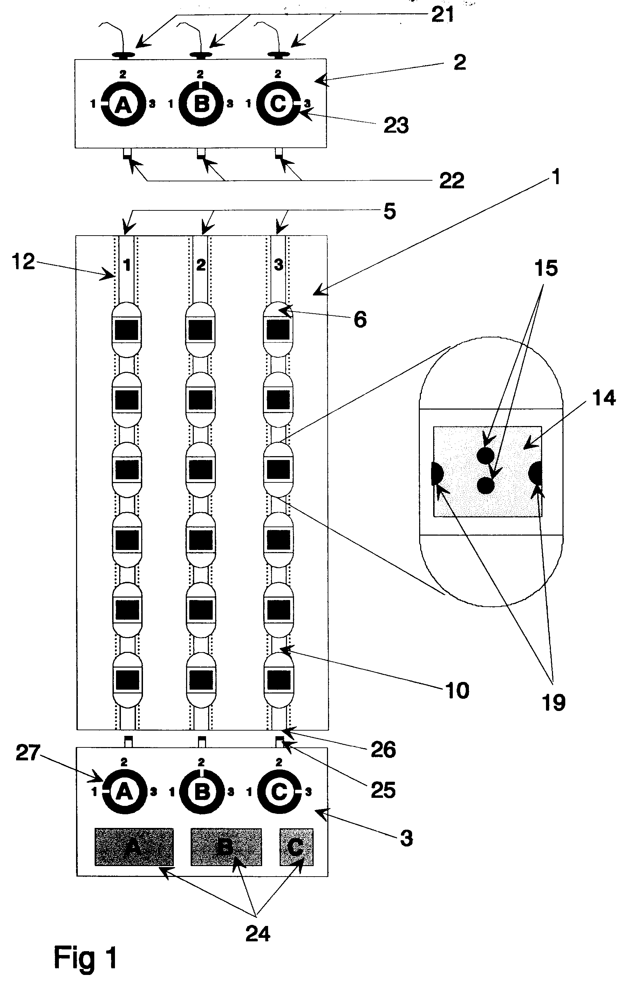

The variants illustrated in Figures 1, 4 and 14-16 include a base plate/stand (1) and normally from 1 to 10, preferably 2 to 4, flow lines (5).

Two or more connecting devices (6) are fitted along each flow line, for connecting modules thereto. For each connecting device, for instance integrated therein, there may be located a part (9) of a flow line which enables the module to be bypassed when its function is deactivated. According to the invention, the most practical embodiments will include at least two and at most 15-25 connecting devices (6) along one and the same flow line (5). The connecting devices are normally spaced apart at regular intervals along a given flow line (5). Each flow line (5) on one and the same base plate (1) will normally have the same number of connecting devices (6). In order to increase the utility of the system, the flow lines will normally extend parallel to each other with the same distance between two adjacently located flow lines.

### The Base Plate

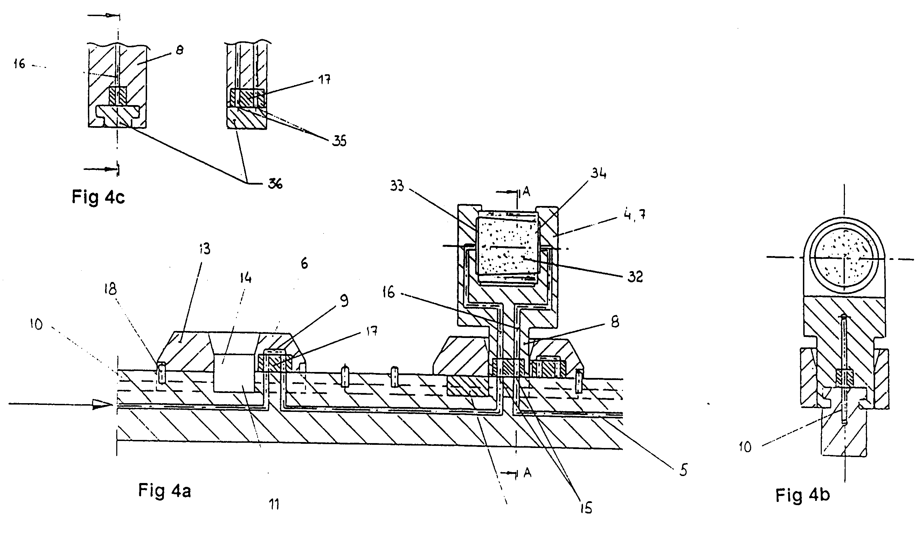

The base plate (1) of the embodiments illustrated in Figures 1 and 4 includes guide means (10) to which the connecting devices (6) are movably fitted. The guide means (10) may have the form of rails or milled grooves which extend along the flow lines (5) between end-positioned connecting devices. The guide means may have a T-shaped cross-section. Alternatively, the guide means may extend perpendicularly to the flow lines and/or each connecting device may have its own guide means. The guide means are normally attached directly to the base plate (1) in the vicinity of their respective flow lines (5). In the case of the embodiments illustrated in Figures 1 and 4, flow lines are built into the guide means (10). The guide means and the base plate may be formed integrally with one another from a single material blank.

As illustrated in Figure 4a, the base plate (1) or a part firmly joined thereto may be provided in the vicinity of the connecting device (6) with a recess (11) which is able to accommodate the bottom part of the end-connecting means (8) of connected modules. The embodiment illustrated in Figure 4a has recesses (11) that are located on the guide means (10).

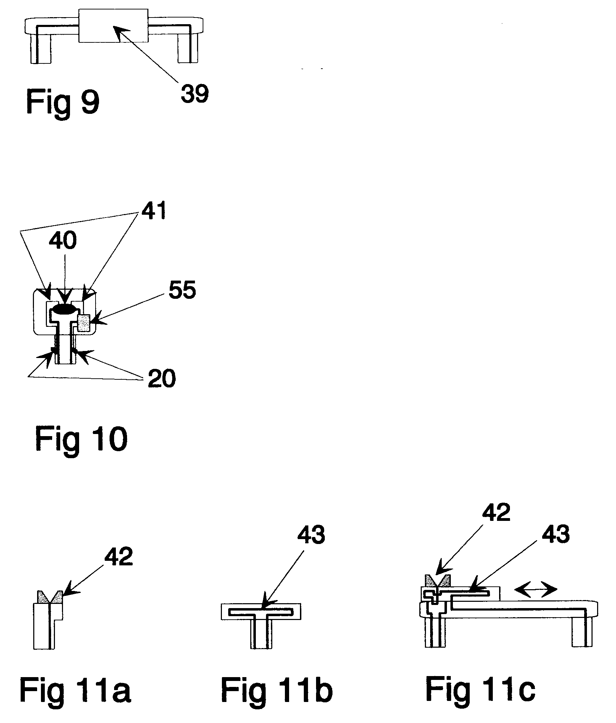

One or more lines/conductors (12) for signal and power transmission from or to connected modules may be arranged in the base plate (1), preferably along the flow lines (5). Transmission may be effected through the medium of optical or electrical signals, or through the medium of pressure changes, etc. The signals may correspond to the values measured in connected detector cells, these signals being transmitted through the lines/conductors (12) to a suitable signal processing unit, where the signals are converted into physical magnitudes. Measured signals may also be used to control a connected module, via the signal processing unit and the lines/conductors (12). The signal processing unit(s) may be a computer which is either incorporated in the system (for instance along lines (12), in modules (see Figure 10), or in connecting devices) or connected externally (not shown in the drawings).

General purpose function units for measuring flow, conductivity, detecting purposes etc may be built in along the flow lines at one or more positions, for instance in the connecting devices or vicinity thereof.

In the case of the embodiment at present preferred, at least three of the connecting devices (6) lie in one and the same plane.

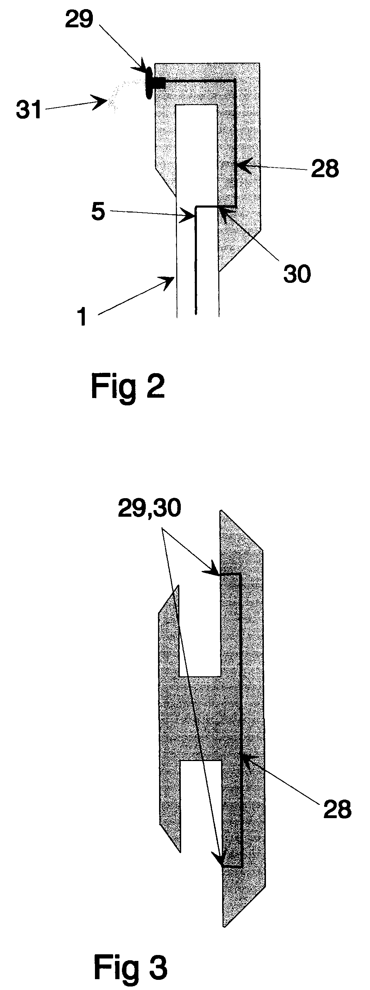

The base plate (1) may be constructed so that several plates can be connected in series or in parallel. Compare Figure 3.

### The Connecting Device

The main parts of the connecting device (6) are comprised of a holder (13) for a module (7), and a valve function for bypassing and/or connecting flow when a module has been placed in the holder (13). In a simple form, the valve function may consist of a tap (not shown in the drawings). A valve function will be described with reference to Figure 4a. The valve function of the Figure 4 embodiment is coupled to a module connecting mechanism. The valve function may also be a separate function which is free from the connecting mechanism.

In the embodiment illustrated in Figures 4a-b, the connecting device (6) includes a holder (13) for a module (4, 7). The holder (13) has a through-penetrating aperture (14) which faces towards the recess (11). The connecting device (6) is movable along its associated guide means (10). When the connecting device is moved along the guide means (10), the flow-line part (9) is disconnected thereby exposing openings (15) in the flow line (5) that in turn become connected to the flow channels (16) of a module placed in the aperture (14). Openings in flow lines and/or their associated channels may be fitted with elastic sealing elements (17), to reduce the risk of leakage.

The guide means (10) may be provided with a so-called positioning device in the form of pins (18), for instance, in order to define a fixed movement for deactivation and/or activation of the connecting device. As illustrated in Figure 4a, the connecting device may have corresponding recesses.

The enlarged part of Figure 1 shows a connecting device from above. The connecting device has been displaced along its guide means (10), so that the openings (15) in the flow line (5) will be seen in the aperture (14) of said device. The means includes contacts (19) for connecting the line/conductor (12) to contacts (20) on a connected module (see Figure 10).

### The End-Pieces

End-pieces (2 and 3), which have mutually different functions can be connected to the flow line on the base plate. The Figure 1 embodiment includes a detachable end-piece (2) which is intended to conduct and distribute flow to the flow lines in an inventive base plate. The end-piece has inlets (21) for supplied liquids (including solutions, buffers, mixtures) and liquid outlets (22). The outlets match the inlets in the base plate. The number of inlets (21) and outlets (22) may be the same or different and may differ from the number of flow lines (5) in the base plate (1). The end-piece (2) includes a control means (23) for mixing and/or guiding incoming flows to predetermined outlets (22) in a predetermined and controllable manner. This control may be manual or automated with programmable control means.

The Figure 1 illustration also includes a detachable end-piece which is intended for connection to the outlet end of a base plate and which enables different sample handling functions (24) to be connected. The end-piece (3) has a number of inlets (25) for connection to the outlets (26) of respective flow lines in the base plate (1). Classic functions can be connected and may possibly also be integrated in the end-piece (3) to a greater or lesser extent. Examples in this regard are functions for waste, collecting fractions and transferring eluate to different external units. The end-piece (3) may be provided with control means (27) for enabling a correct flow line (5) to be guided to a desired sample handling function. This control of the end-piece (3) may be analogous with the control of the end-piece (2).

Figure 2 illustrates a method of connecting the end-pieces. The end-piece of this embodiment includes a flow channel (28) having an inlet (29) and an outlet (30) enabling the connection of one or more external tubing (31). The end-piece is configured so as to enable it to be fitted over one end of the base plate (1), so that its outlet (30) is connected functionally to one of the flow channels (5) of the base plate, with the aid of the guide means (10). The end-piece may contain the same functions as the end-piece of the Figure 1 embodiment. The end-piece shown in Figure 2 intended for base plates according to Figure 1 may be provided with milled grooves or with rails which match the guide means (10).

Figure 3 illustrates a connecting piece for connecting together two base plates. The principle of connecting to a base plate is the same as that illustrated in Figure 2.

### Modules Described with a Starting Point from a Separation Module

Figures 4a-c illustrate an embodiment of a separation module (4) which contains a separation medium (32), flow channels (16), a flow spreading/collecting function (33 and 34 respectively) and an end-connecting means (8). When the module is not used, the openings (35) in the module flow channels are normally covered by closure means (36) to avoid air bubbles and dehydration. In the illustrated embodiment, the closure device (36) is T-shaped and fits into a T-shaped groove milled in the end-connecting means. When the closure device is displaced sideways, openings (35) in the flow channels are exposed. The openings (35) will preferably be surrounded by a sealing element (17) which presses against the closure device (36).

The outer shape of the end-connecting means (8) are the same for modules having different functions when they are to be used on the same base plate. By "the same" is meant that they fit a plurality of holders (13) in one and the same inventive system. In a connected state, the configuration of the flow channel openings (35) matches the openings (15) in the flow line (5), so as to enable flow to pass through.

Figures 4a-c illustrate a variant of possible module connection functions. When the module is placed in the aperture (14), the closure device (36) and the T-shaped milled groove of the end-connecting means (8) are located on the bottom of the recess (11), the recess is formed so that no space in form of play will be found between the closure device and the bottom of the recess. When a module (4) is inserted in the connecting device (6) and said device is moved along the guide means (10), the milled T-groove of the end-connecting means matches the T-shape of the T-shaped rail of the guide means, which means that the closure device (36) remains in the recess (11) and the openings (35) of the flow channels are exposed. Upon completion of this movement, the openings (35) match corresponding openings (15) in the flow line (5). The T-shape of the guide means (10) and the milled groove in the end-connecting means fix the module in the holder (14). When movement is completed, liquid is able to flow into the module and, when appropriate, may be returned to one of the existing flow lines (5). The sealing element (17) presses resiliently against the upper surface of the guide means, to provide a sealing effect.

When the connecting device (6) is moved back to its original position, so as to disconnect the module (4), the closure device (36) is caught by the T-groove milled in the end-connecting means, thereby closing the module. The flow of liquid is caused to bypass the module, through the flow-line part (9).

### Types of Modules

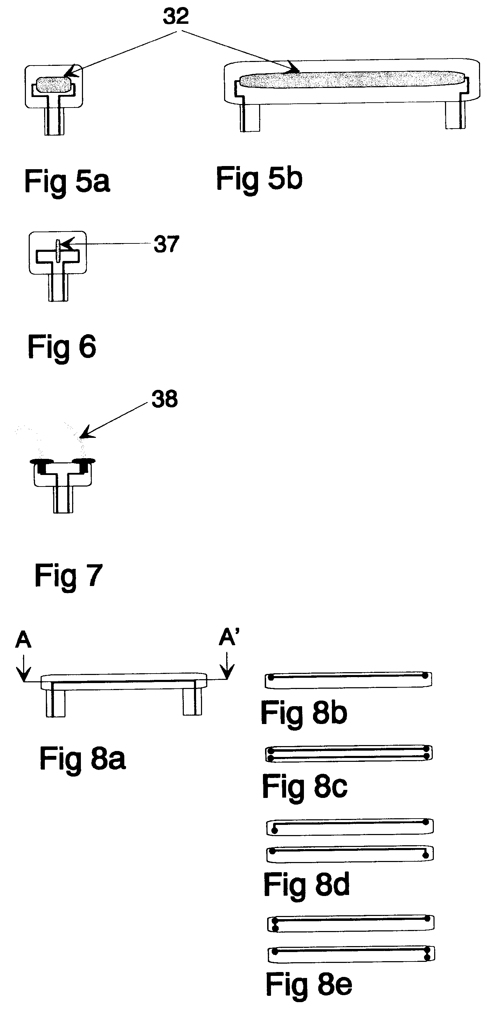

Figures 5-12 illustrate different types of modules. A common feature of the illustrated modules is that they have one or more, preferably 1-2 end-connecting means with associated flow channels and a functional part through which liquid shall flow, be mixed, branched-off or stopped. The flow channels are emphasized in the Figures by heavy black lines. The end-connecting means of respective modules are shown schematically. The requirements for a geometric shape that is adapted to the connecting device and flow channels are the same as those mentioned above with respect to a separation module. The modules may be provided with closure device analogous with that described with reference to Figure 4.

Figures 5a and 5b illustrate separation modules which include a separation medium in the form of a matrix (32) and flow channels. The module illustrated in Figure 5a has an inlet and an outlet through a common end-connecting means, and the module illustrated in Figure 5b through separate ends. The end pieces of the separation medium have spreading and collecting functions respectively, which can be achieved with the aid of filter paper inserts, end-piece abutment surfaces with inlets combined with systems of channels, etc. (33 and 34 in Figure 4a). The matrix (32) may be comprised of discrete, packed particles of cross-linked polysaccharide, polyacrylamide, and the like, or may be continuous (monolithic), i.e. have the form of a porous body. The matrix may exhibit substituents enabling the desired type of chromatography to be run (e.g. ion exchange groups, hydrophobic groups, affinity ligands). The width and the length of the matrix are determined by the separation performance desired. The matrix may be given the form of a membrane. It is believed that the future preferred embodiments of the invention will comprise matrixes in form of a continuous body with a cylindrical or frusto conical shape, narrowing in the flow direction of the column (see Figures 4a-b).

Figure 6 illustrates a filter module which includes flow channels having an inlet and an outlet, a common end-connecting means and a filter (37). The filter may consist of material conventional for chromatography. Although not shown, filter modules may also have two end-connecting means.

Figure 7 illustrates a connecting module. The module has an end-connecting means and two separate flow channels, each provided with an inlet and an outlet. External units for sample handling, detection, etc., can be connected to the module by means of tubings (38). Flow from a connected external unit can be passed back to the module and from there to the flow line of the base plate through one of the tubings. This type of module may be provided with two or more end-connecting means (not shown).

Figure 8a illustrates a flow bridge which is intended to conduct liquid flow from one connecting device to another. Flow bridges may also have three or more end-connecting means in order to bridge to more than two flow lines/connecting devices.

Figures 8b-e illustrate different variants of the bridge shown in Figure 8a and show how the bridge can be configured to conduct and distribute flow between the flow lines in the base plate. The bridges are shown in a sectional view taken on the line A-A' in Figure 8a. The solid rings symbolize an inlet or outlet coming from beneath. The bridges illustrated in Figure 8e include the possibility of mixing or dividing flow.

Figure 9 illustrates a bridge where the rectangle (39) symbolizes that a flow bridge may incorporate an optional function in the form of valves, filter, matrices, additional connection, detectors, etc.

Figure 10 illustrates a detector module. The detector unit (40) may be based on pH, UV, IR, conductivity, capacitance, refractive index, etc. The transmission of signals from the module is effected through lines/conductors (41) and contacts (20) to corresponding contacts (19 - shown in Figure 1) in the connecting device. Correspondingly, other modules (for instance valve modules) may be provided with power and signal transmission lines/conductors. Detector modules may be equipped with signal processing units (55).

Figures 11a-c illustrate modules that can be used in conjunction with sample application. Figure 11a illustrates a simple sample injection module which includes a through-passing flow channel with an injection port (42) at one end thereof. Figure 11b illustrates a sample loop module which includes a loop (43), an end-connecting means and a flow channel. Modules according to Figures 11a and 11b are preferably used together. These modules can be connected consecutively, one after the other, along one and the same flow line, whereafter the loop (43) can be filled by injecting sample through the port (42). Subsequent to being filled, the loop (43) can be moved to an appropriate position in the system set-up. Figure 11c illustrates a bridge module having an injection port (42) and a loop which can be moved laterally (as shown by the double-headed arrow). Figure 11c shows the loop (43) in a sample-charging position. The loop is filled as sample is injected. Surplus sample is passed to the right end-connecting means. As the loop is moved sideways to the left (the injection position), it is connected to the left end-connecting means and the sample can be passed into the flow line connected to this end. Displacement of the loop creates a valve function which may, in principle, be constructed in a similar fashion to the valve function in Figure 4.

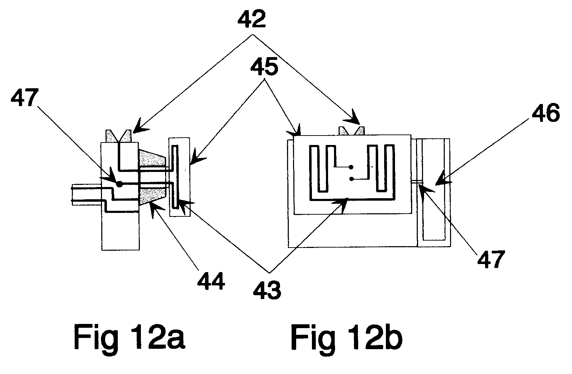

Figures 12a and 12b illustrate a module having a connecting device (44) for a sample loop module (45). The module also includes an injection port (42), a vessel for waste (46), flow channels and an end-connecting means. The module illustrated in Figure 12b is a front view of the module illustrated in Figure 12a. The connecting device (44) may be constructed analogously with what has been described with reference to Figures 4a-b. The sample loop module may be the same as that shown in Figure 11b. Figures 12a-b show the loop in its charging position. The loop (43) is filled by injecting sample through the port (42), and excess sample is passed to the waste vessel (46) through the channel (47). When the connecting means (44) with a connected loop is displaced downwards, the loop is connected functionally to the flow channels in the end-connecting means of the module (the injection position). The connection to the waste vessel is disconnected at the same time. In the injection position, the loop contents can be passed to the flow line to which the end-connecting means is coupled.

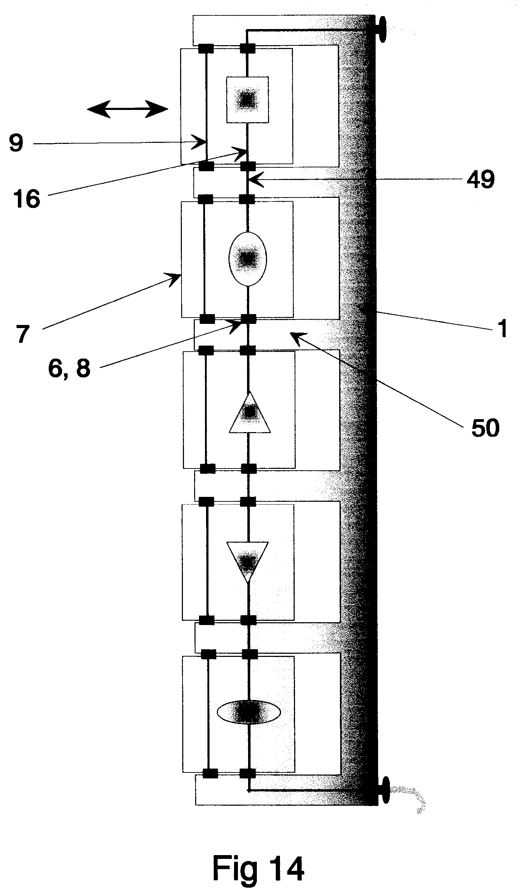

Figures 12a-b show that a module may have both an end-connecting means and a connecting device. The principle can be applied to modules having other functions and enables the inventive system to be extended in several dimensions without the aid of bunches of tubings. Compare Figures 14-16.

A so-called stop module, i.e. a module which completely lacks a flow channel through the end-connecting means is one example of such additional modules.

One and the same module may include two or more of the functions mentioned in connection with Figures 5-12. Thus modules carry at least one function selected from the group consisting of separation, filtration, connector for one or more external unit(s), valve, conductor for liquid flow between two or more flow lines/connecting devices, injection port for liquids, loop for samples, detector and stop.

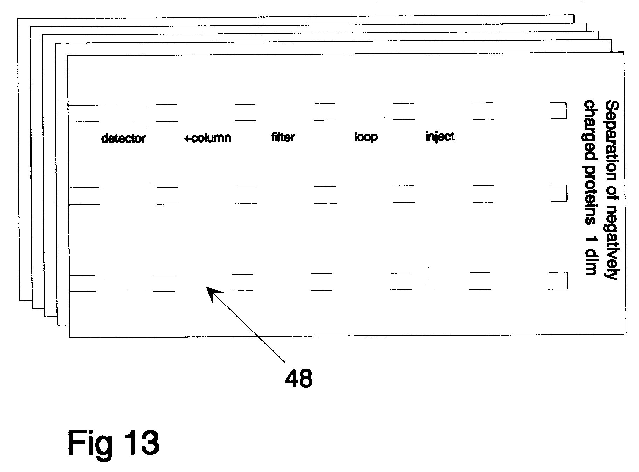

Figure 13 illustrates a stack of installation templates for the embodiment according to Figure 1. The templates are preferably in the form of sheets having openings (48) for accommodating the connecting device of the base plate. Each opening discloses the type of module to be connected-up. Connection of a sequence is effected simply by placing the template over the base plate and then connecting-up the modules. To assist people with defective vision, the openings may be provided with detectors which produce an alarm when the wrong module is placed in an opening. Alternatively, those openings which shall not be used may be closed with a padlock or some other type of barrier.

Figures 14-16 illustrate principles of further embodiments of the invention. Different functions of the modules shown are illustrated with the aid of centrally positioned rectangles, ovals, triangles, etc. The functions may be the same type of functions as those described above. Flow lines and flow channels have been shown in heavy (bold) lines. Solid black triangles represent valve functions that divide or join flows. As with the embodiments according to Figures 1 and 4, connecting means and end-connecting means shall match one another geometrically and functionally. The connecting devices and/or end-connecting means illustrated may also be provided with closure devices in analogy to the embodiment given in Figures 4a-c.

Figure 14 illustrates a variant of the invention which includes a stand or a base plate (1) in which a flow line segment (49) is fixedly mounted. The modules (7) can be connected-up by moving the modules in the direction of the double-headed arrow. The modules have two flow channels (9 and 16). The channel (9) forms a flow bypass and the channel (16) connects the chromatography function of the module to the fixed segments (49). The flow-line segments (49) and the flow channels (9) correspond to the flow line (5) in Figures 1 and 4.

Connecting devices/end-connecting means, including sealing elements, are shown in Figure 14 as solid rectangles (6 and 8). Movement of the modules in the aforedescribed manner results in a valve function. The outwardly projecting parts (50) may be detachable, so as to provide room for longer modules, for instance longer separation modules.

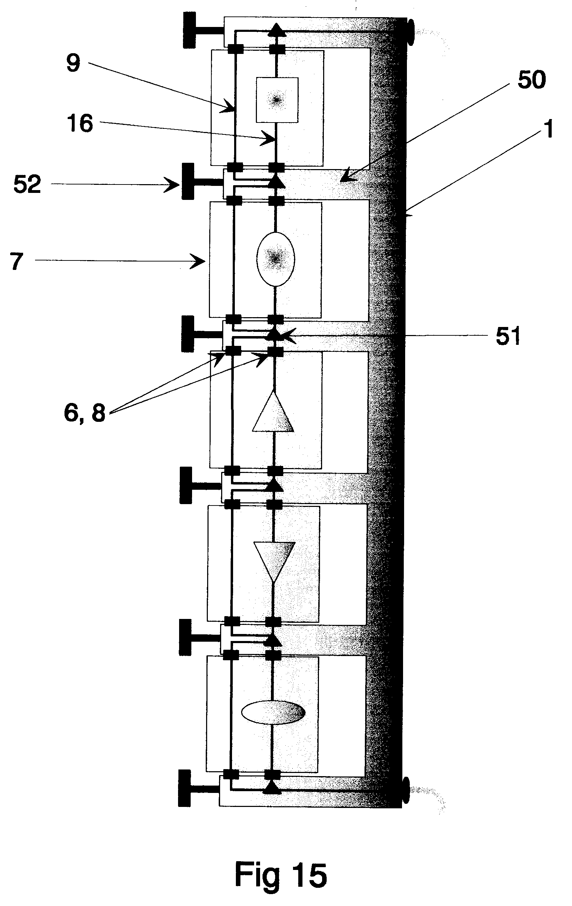

Figure 15 is a variant of the invention which includes a stand or a base plate (1) in which there is mounted flow-line segments in outwardly projecting parts similar to Figure 14 (49 and 50) having valve functions (51) which can be controlled manually by control means (52) or automatically with the aid of a computer (see the aforegoing with regard to Figures 1 and 4). The modules (7) can be connected-up, by moving the modules in the manner described with reference to Figure 14. The modules have two flow channels (9 and 16). The channel (9) serves to bypass the flow while the channel (16) connects the chromatography function of the module to the flow line segment (49). Correspondence to the flow line (5) in Figures 1 and 4 is found in the flow-line segments (49) in the outwardly projecting parts (50) and the flow channels (9). Connecting means/end-connecting means, including any sealing elements, are illustrated in the Figure by solid rectangles (6 and 8). Similar to the Figure 14 embodiment, the outwardly projecting parts (50) may be detachable.

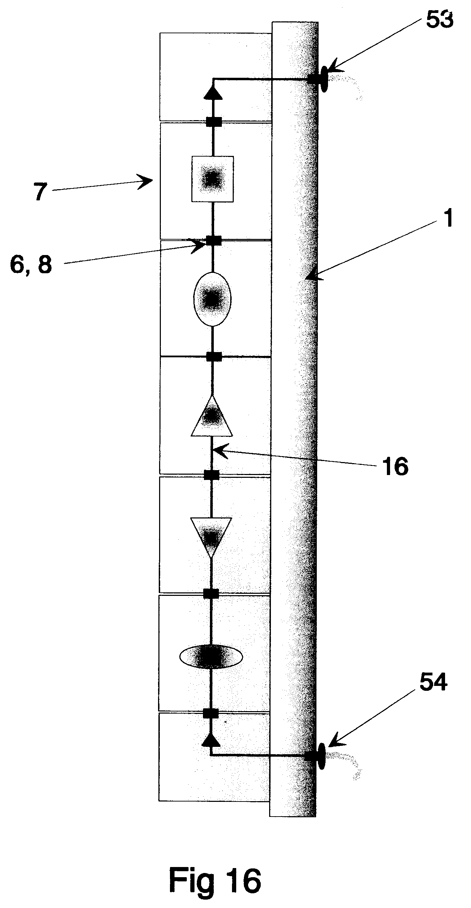

Figure 16 illustrates a variant in which the modules (7) are detachably connected to a base plate which includes supply flow and exhaust flow (53 and 54 respectively). The flow line between the supply flow and exhaust flow is defined by the flow channels of the mutually connected modules. Connecting devices/end-connecting means, including any sealing elements and possible valve functions, are illustrated in the Figure by solid rectangles (6 and 8).

## Claims

1. A liquid chromatographic system comprising
(a) at least one chromatography module (4);
(b) at least one flow line (5); and
(c) at least one ancillary module (7) which is not a separation module,
**characterized** in that the modules (4 and 7) have functionally similar end-connecting means (8); in that two or more functionally similar connecting devices (6) are connected to the flow line (5) and provided for connecting said modules (4 and 7) to the flow line, the end-connecting means (8) and the connecting devices (6) being such that any of said modules (4, 7) can be connected to any of said connecting devices (6) on said flow line (5); and in that the system additionally comprises a base plate carrying the flow line or segments thereof, in which said modules are connected to each other by segments of the flow line.

2. A system according to Claim 1, **characterized** in that the connection between said connecting device (6) and said end-connecting means (8) is a quick-coupling type connection.

3. A system according to any one of Claims 1-2, **characterized** in that said at least one ancillary module (7) has at least one function selected from the group consisting of connector for external units, valve, conductor for liquid flow between two or more flow lines/connecting devices, injection port for liquid, loop for samples, detecting and stop.

4. A system according any one of Claims 1-3, **characterized** in that said at least one chromatography module (4) has solely one end-connecting means (8) with flow channels (16) for conducting flow from and to a separation medium (32) placed in the chromatography module.

5. A system according any one of Claims 1-3, **characterized** in that said at least one chromatography module (4) has solely two end-connecting means (8) with flow channels (16) for conducting flow from and to a separation medium (32) placed in the module.

6. A system according to any one of Claims 1-5, **characterized** in that there are conductors (12) for signal and power transmission to connected modules.

7. A system according to claim 6, **characterized** in that the system furthermore comprises one or more signal processing unit(s).

8. A system according to any of claims 1-7, **characterized** in that there is a flow line part (9) which is connected to each respective end-connecting means and which allows liquid by-pass of a deactivated module.

## Patentansprüche

1. Chromatographisches Flüssigkeitssystem, umfassend
(a) wenigstens ein Chromatographiemodul (4);
(b) wenigstens eine Strömungsleitung (5); und
(c) wenigstens ein Zusatzmodul (7), welches kein Trennmodul ist,
dadurch **gekennzeichnet,** daß die Module (4 und 7) funktionell gleichartige bzw. ähnliche Endverbindungsmittel (8) haben; daß zwei oder mehr funktionell gleichartige bzw. ähnliche Verbindungseinrichtungen (6) mit der Strömungsleitung (5) verbunden und für das Verbinden der Module (4 und 7) mit der Strömungsleitung (5) vorgesehen sind, wobei die Endverbindungsmittel (8) und die Verbindungseinrichtungen (6) derart sind, daß jedes der Module (4, 7) mit jeder der Verbindungseinrichtungen (6) auf bzw. in der Strömungsleitung (5) verbunden werden kann; und daß das System zusätzlich eine Basisplatte umfaßt, welche die Strömungsleitung oder Abschnitte davon trägt, worin die Module miteinander durch Abschnitte der Strömungsleitung verbunden sind.

2. System gemäß Anspruch 1, dadurch **gekennzeichnet,** daß die Verbindung zwischen der Verbindungseinrichtung (6) und dem Endverbindungsmittel (8) eine Verbindung vom Schnellkupplungs- bzw. -verbindungstyp ist.

3. System gemäß irgendeinem der Ansprüche 1 bis 2, dadurch **gekennzeichnet,** daß wenigstens ein Zusatzmodul (7) wenigstens eine Funktion hat, die aus der Gruppe ausgewählt ist, welche aus Verbinder für externe Einheiten, Ventil, Leiter für Flüssigkeitsströmung zwischen zwei oder mehr Strömungsleitungen/Verbindungseinrichtungen, Injektionskanal- bzw. -durchlaß für Flüssigkeit, Schleifen für Proben, Detektion und Stop besteht.

4. System gemäß irgendeinem der Ansprüche 1 bis 3, dadurch **gekennzeichnet,** daß das wenigstens eine Chromatographiemodul (4) allein ein Endverbindungsmittel (8) mit Strömungskanälen (16) zum Leiten von Strömung von und zu einem Trennmedium (32), das in dem Chromatographiemodul plaziert ist, hat.

5. System gemäß irgendeinem der Ansprüche 1 bis 3, dadurch **gekennzeichnet,** daß das wenigstens eine Chromatographiemodul (4) allein zwei Endverbindungsmittel (8) mit Strömungskanälen (6) für das Leiten von Strömung von und zu einem Trennmedium (32), das in dem Modul plaziert ist, hat.

6. System gemäß irgendeinem der Ansprüche 1 bis 5, dadurch **gekennzeichnet,** daß Leiter (12) für Signal- und Leistungsübertragung zu verbundenen Modulen vorhanden sind.

7. System gemäß Anspruch 6, dadurch **gekennzeichnet,** daß das System weiterhin eine oder mehrere Signalverarbeitungseinheit(en) umfaßt.

8. System gemäß irgendeinem der Ansprüche 1 bis 7, dadurch **gekennzeichnet,** daß eine Strömungsleitung teilweise vorhanden ist, welche mit jedem jeweiligen Endverbindungsmittel verbunden ist und welche es der Flüssigkeit ermöglicht, ein entaktiviertes Modul zu umgehen.

## Revendications

1. Système de chromatographie en phase liquide comportant
(a) au moins un module (4) de chromatographie ;
(b) au moins une conduite d'écoulement (5) ; et
(c) au moins un module auxiliaire (7) qui n'est pas un module de séparation,
caractérisé en ce que les modules (4 et 7) ont des moyens de raccordement extrêmes fonctionnellement similaires (8) ; en ce que deux ou plus de deux dispositifs de raccordement (6), fonctionnellement similaires, sont raccordés à la conduite d'écoulement (5) et prévus pour un raccordement desdits modules (4 et 7) à la conduite d'écoulement, les moyens de raccordement extrêmes (8) et les dispositifs de raccordement (6) étant tels que l'un quelconque desdits modules (4, 7) peut être raccordé à l'un quelconque desdits dispositifs de raccordement (6) sur ladite conduite d'écoulement (5) ; et en ce que le système comporte en outre une plaque de base portant la conduite d'écoulement ou des segments de celle-ci, dans laquelle lesdits modules sont raccordés entre eux par des segments de la conduite d'écoulement.

2. Système selon la revendication 1, caractérisé en ce que le raccordement entre ledit dispositif de raccordement (6) et lesdits moyens de raccordement extrêmes (8) est un raccordement du type à accouplement rapide.

3. Système selon l'une quelconque des revendications 1-2, caractérisé en ce que ledit ou chaque module auxiliaire (7) possède au moins une fonction choisie dans le groupe constitué d'un raccord pour des unités extérieures, d'une vanne, d'un conduit pour l'écoulement d'un liquide entre deux ou plus de deux conduites d'écoulement/dispositif de raccordement, d'un orifice d'injection pour un liquide, d'une boucle pour des échantillons, d'une détection et d'une obturation.

4. Système selon l'une quelconque des revendications 1-3, caractérisé en ce que ledit ou chaque module (4) de chromatographie ne comporte qu'un moyen (8) de raccordement extrême avec des canaux d'écoulement (16) pour conduire un écoulement depuis et vers un milieu de séparation (32) placé dans le module de chromatographie.

5. Système selon l'une quelconque des revendications 1-3, caractérisé en ce que ledit ou chaque module (4) de chromatographie ne comporte que deux moyens de raccordement extrêmes (8) avec les canaux d'écoulement (16) pour conduire un écoulement de et vers un milieu (32) de séparation placé dans le module.

6. Système selon l'une quelconque des revendications 1-5, caractérisé en ce qu'il y a des conducteurs (12) pour la transmission de signaux et de puissance à des modules raccordés.

7. Système selon la revendication 6, caractérisé en ce que le système comporte en outre une ou plusieurs unités de traitement de signaux.

8. Système selon l'une quelconque des revendications 1-7, caractérisé en ce qu'il y a une conduite d'écoulement qui est partiellement raccordée à chaque moyen de raccordement extrême respectif et qui permet à un liquide de contourner un module désactivé.
